# EUROPEAN PATENT SPECIFICATION

(11) **EP 1 888 166 B1**
(45) Date of publication and mention of the grant of the patent: **30.03.2016**
(21) Application number: 06784737.6
(22) Date of filing: 09.06.2006
(51) Int. Cl.: A61M 1/10, H02K 33/12, A61M 1/12, H01F 7/10

(54) **SINGLE SAC VENTRICULAR ASSIST DEVICE**
VENTRIKULÄRES HILFSSYSTEM MIT EINZELSACK
DISPOSITIF D'ASSISTANCE VENTRICULAIRE A SAC UNIQUE

(30) Priority: 09.06.2005 US 689617 P
(43) Date of publication of application: 20.02.2008
(73) Proprietor: World Heart Corporation, Oakland, California 94621 (US); Jassawalla, Jal, Orinda, California 94563 (US); Miller, Phillip J., California 94708 (US); Laforge, David H., Kensington, California 94708 (US)
(72) Inventor: JASSAWALLA, Jal, Orinda, California 94563 (US); MILLER, Phillip J., California 94708 (US); LAFORGE, David H., Kensington, California 94708 (US)
(74) Representative: Sattler de Sousa e Brito, Clara
(86) International application number: PCT/US2006/022644
(87) International publication number: WO 2006/135822

(56) References cited:
- WO-A2-2004/020022
- DE-A1- 3 804 768
- DE-C1- 19 609 281
- US-A- 2 323 441
- US-A- 3 842 440
- US-A- 5 607 292
- US-A1- 2004 097 782
- US-A1- 2004 116 769
- US-B1- 6 264 601
- US-B1- 6 284 601
- US-B1- 6 406 422

## Description

### FIELD OF THE INVENTION

The present invention relates to devices for pumping blood. More particularly, the present invention relates to ventricular assist devices (VADs) utilized to assist or replace the function of one or both ventricles of the heart.

### BACKGROUND OF THE INVENTION

The American Heart Association estimates that there are approximately 5 million people with congestive heart failure in the United States and 550,000 new cases diagnosed annually. Those numbers will only rise in the foreseeable future with the aging of the baby-boom generation. According to the Framingham Heart Study, the five-year mortality rate for patients with congestive heart failure was 75 percent in men and 62 percent in women. Standard medical and surgical therapies benefit only a small percentage of patients with ventricular dysfunction. Potential cardiac transplant recipients with hemodynamic instability may receive temporary mechanical circulatory support, such as an implantable blood pump, as a bridge to cardiac transplantation. Moreover, estimates in the field suggest that 17,000 to 66,000 patients each year in the United States may benefit from a permanent blood pump.

The ventricular assist device (VAD) is a blood pump designed to assist or replace the function of either ventricle, or both ventricles, of the heart. A right ventricular assist device (RVAD) supports pulmonary circulation by receiving or withdrawing blood from the right ventricle and returning it to the pulmonary artery. A left ventricular assist device (LVAD) supports systemic perfusion by receiving or withdrawing blood from the left ventricle (or left atrium) and returning it to the aorta. A biventricular assist device (BVAD) supports both ventricles of the heart. Ventricular assist devices may be either implantable or extracorporeal, with implantable VADs positioned intracorporeally in the anterior abdominal wall or within a body cavity (other than the pericardium) and with extracorporeal VADs located paracorporeally, along the patient's anterior abdominal wall, or externally at the patient's bedside.

The first ventricular assist devices attempted to mimic the pulsatile flow of the natural left ventricle (LV) by utilizing flexible chambers with volumes approximately equal to the volume of the respective ventricle being assisted. The typical volume of blood expelled by the left ventricle of an adult is between 70-90 ml, but may range from 40-120 ml. The chambers are expanded and contracted, much like a natural ventricle, to alternately receive and expel blood. One way valves at the inlet and outlet ports of the chambers ensured one way flow therethrough.

So-called "pulsatile pumps" may include one or a pair of driven plates for alternately squeezing and expanding flexible chambers. The flexible chambers typically comprise biocompatible segmented polyurethane bags or sacs. The blood sac and drive mechanism are mounted inside a compact housing that is typically implanted in the patient's abdomen. A controller, backup battery, and main battery pack are electrically connected to the drive mechanism. Even the most basic drive mechanisms of the prior art are relatively complex and expensive, and typically incorporate some type of mechanical cam, linkage, or bearing arrangement subject to wear.

Because of the varying volume of the blood sac within the rigid encapsulation housing of pulsatile pumps, accommodation must be made for the air displaced thereby. Some devices utilize a percutaneous tube vented to the atmosphere, which is a simpler approach but involves skin penetration. Another possible approach for fully-implantable VAD systems is to use a volume compensator. This is a flexible chamber, implanted in the thoracic cavity adjacent to the lungs and communicating with the air space within the housing and outside the blood sac via an interconnecting tube. As the blood sac expands with incoming blood, air is displaced from the housing to the volume compensator. Conversely, expulsion of blood from the blood sac creates a negative pressure within the housing and pulls air from the volume compensator. While eliminating the infection risk due to the skin penetration of the percutaneous vented tube, the volume compensator poses certain challenges: increased system complexity, an additional implanted component and potential site of infection, maintaining long-term compliance of the implanted volume compensator sac, problems associated with gas diffusion in or out of the enclosed volume, and problems associated with changes in ambient pressure, such as experienced during a plane flight.

One example of an electric pulsatile blood pump is the Novacor N100 Left Ventricular Assist System (World Heart Inc., Oakland, Calif.). This system contains a single polyurethane blood sac with a nominal stroke volume of 70 ml that is compressed by dual symmetrically opposed pusher plates in synchronization with the natural left ventricle contraction. The pusher plates are actuated by a spring-decoupled solenoid energy converter. The blood pump and energy converter are contained within a housing that is implanted in the patient's abdomen. The N100 employs a percutaneous vent tube that also carries power and control wires.

An example of an electric pulsatile blood pump not requiring external venting is disclosed in U.S. Patent No. 6,264,601 ("the '601 patent"). The system of the '601 patent has two pumping chambers formed from two flexible sacs separated by a pusher plate, with the sacs and pusher plate contained within one housing. A electromagnetic drive system acts on an iron armature surrounded by a cylindrically symmetric permanent magnet within the pusher plate to alternatively pump blood through the two sacs by compressing one sac and then the other against the housing. The electromagnetic drive is also referred to herein as the direct magnetic drive (DMD). Since each sac contains only fluid that is alternately received and discharged as the pusher plate reciprocates, the total volume of the pump remains constant during pumping and no venting or volume compensator is required. The input and output of each sac includes a one-way valve, providing unidirectional flow that pumps the fluid in a preferred direction. The most efficient use of the electromagnetic drive system is achieved when the power and energy required in each pump stroke is approximately equal.

The '601 patent describes several alternative arrangements for using a blood pump, including a left or right VAD that couples the input and output flows from each chamber in either parallel or series, and a BVAD that separately uses two separate VADs to assist the left and right ventricle. One embodiment described in the '601 patent is a series-displacement pump, in which a first chamber receives a fluid for pumping, and provides that fluid to the input of a second chamber for further pumping ("the '601 series-displacement pump"). In operation, the '601 series displacement pump alternates between a pump stroke and a transfer stroke. When used as a VAD, the pump stroke pumps blood from the second chamber into the aorta while blood is drawn from the ventricle into the first chamber. In the transfer stroke, blood from first chamber is transferred to the second chamber. The fluid connection between the chambers is an external transfer conduit that connects the output of the first sac to the input of the second sac.

The '601 series-displacement pump has several advantages over other prior art pumps including, but not limited to, the ability to provide pulsatile flow, the use of fewer blood conduits and valves, and reduced size. However, the electromagnetic drive system of the '601 patent is optimized for bi-directional use, while the power and transfer strokes of the '601 series-displacement pump each have different power and energy characteristics. While the pump of the '601 patent is capable of operating as a series-displacement pump, there are energy losses that result from not having the drive and pump matched for series operation. Also, in general, the pump of the '601 patent includes a permanent magnet to drive the pusher plates that has a radially symmetric design that is expensive and difficult to manufacture.

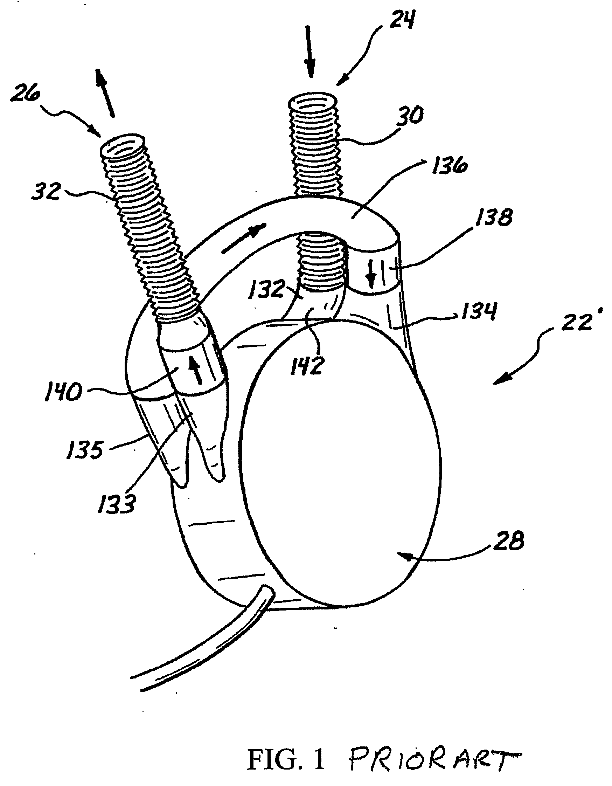

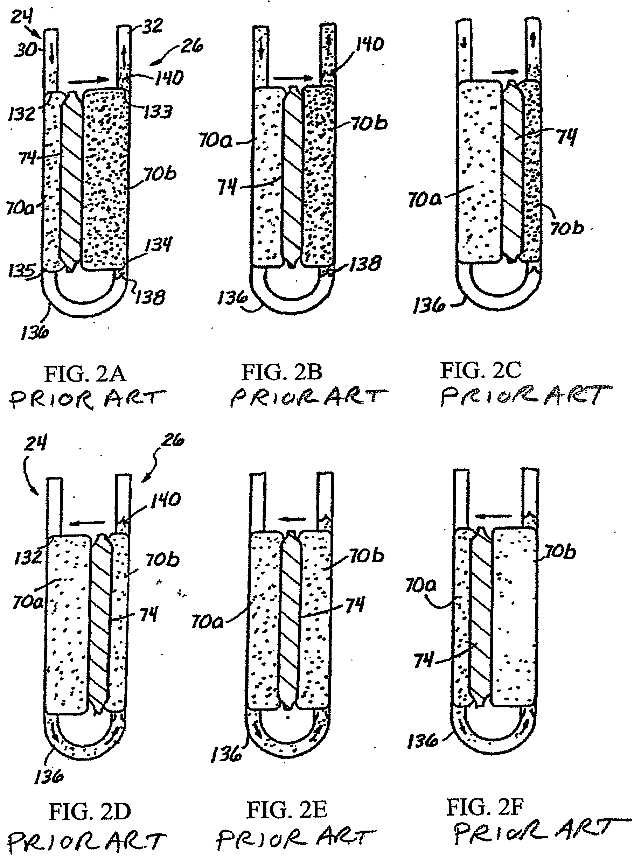

A Pump/Drive Unit (PDU) is one of the configurations of the DMD described in the '601 patent as the "Series-displacement VAD". As described in the '601 patent, a pump 28 is configured in a ventricular assist system 22' shown in FIGs. 1 and 2A-2F in which the chambers 70 are connected in series. A flexible inlet segment 30 of the inlet conduit 24 connects to, for example, the left ventricle of the heart, not shown, and the flexible outlet segment 32 of the outlet conduit 26 connects to, for example, the aorta, not shown. In this embodiment, the flexible inlet segment 30 is only connected to the inlet port 132 of one of the chambers, such as the left chamber 70a, and the flexible outlet segment 32 is only connected to the outlet port 133 of the other chamber, such as the right chamber 70b.

According to the embodiment shown in FIG. 1, a transfer conduit 136 is connected between the outlet port 135 of the chamber connected to the flexible inlet segment 30 (the left chamber 70a) and to the inlet port 134 of the chamber connected to the flexible outlet segment 32 (the right chamber 70b). In addition, a pair of valves are provided, including an inlet valve 138 disposed at the inlet port 134 of the chamber connected to the outlet conduit 26 and an outlet valve 140 disposed at the outlet port 133 of the chamber connected to the outlet conduit 26.

In accordance with the series flow blood pump 28 exemplified in FIG. 1, blood from the left ventricle is initially pumped to the left chamber 70a in the inlet conduit 24. Coils, not visible in the views of FIGs. 1-2F, are activated to move the plate 74 to the right as shown by the arrow in FIG. 2A, thereby ejecting blood received within the right chamber 70b through the outlet port 133 and the outlet valve 140 and into the flexible outlet segment 32 for delivery to the aorta. During this ejection stroke of the plate 74, the inlet valve 138 prevents blood from entering the transfer conduit 136. In addition, the left chamber 70a is expanded, thereby drawing oxygenated blood through the inlet conduit 24 from the left ventricle (not shown) into the left chamber as shown in FIG. 2B. At the end of the ejection stroke as shown in FIG. 2C with the plate 74 positioned to the right, the left chamber 70a is filled with oxygenated blood from the left ventricle, and the right chamber 70b is compressed to a minimum volume.

The coils are then activated to move the plate 74 to the left as shown by the arrows in FIGS. 2D and 2E, thereby drawing blood from the left chamber 70a into the right chamber 70b via the transfer conduit 136. The outlet valve 140 prevents blood in the aorta or the outlet conduit 26 from being drawing back into the right chamber 70b. In addition to left ventricular pressure, the low pressure within the right chamber 70b caused by the expansion of the chamber ensures that blood within the left chamber 70a enters the right chamber 70b and is not ejected back into the inlet conduit 24. If desired, an additional valve may be disposed at the inlet port 132 of the left chamber 70a to also prevent blood from entering the inlet conduit 24. At the end of the transfer stroke as shown in FIG. 2F with the plate 74 positioned to the left, the right chamber 70b is filled with oxygenated blood from the left ventricle, and the left chamber 70a is compressed to a minimum volume. The ejection stroke illustrated in FIGS. 2A-2C and the transfer stroke illustrated in FIGS. 2D-2F may be repeated in accordance with the exemplary methodology of the invention described above. Control of the system 22' may be based on the sensed input pressure. An advantage of the ventricular assist system 22' is the reduction of the number of valves needed, from four to two. This in turn reduces the cost of the device.

Thus, the configuration illustrated in FIGs. 1 - 2F transfers blood between the two pumping chambers, and ejects into the aorta only from the second chamber. The configuration therefore requires only two valves, rather than four, and avoids the complication of bifurcated conduits. The pumping chamber shown in FIG. 1 ejects into the aorta through the outflow valve, while the "pre-chamber" sac, at the left in FIG. 1, serves as the displacement chamber and communicates with the left ventricle via a non-valved conduit. Since this VAD functions with no vent or compliance device, its total volume is constant so the inflow and outflow are always equal.

The known blood pump, VAD, shown in FIGs. 1-2F has two pumps, corresponding dual chambers and sacs, a transfer conduit between chambers of each pump, and a corresponding valve housing. A drawback of this known device is that having two pumps along with the corresponding transfer conduit and valve housing adds complexity and size to the device and to the electronics for controlling it. What is needed therefore is a VAD having only one pump that substantially reduces the complexity and size associated with known two pump, dual-chamber devices. What is also needed is for the VAD to have a bearingless electromagnetic drive and simpler electronics to reduce size and complexity and increase reliability. What is also needed is a single sac VAD that permits slower ejection to allow a smaller vent tube to reduce size and the risk of infection. What is needed, therefore, is a simpler, more efficient single sac ventricular assist device of reduced size, weight, and complexity to facilitate implantation and use of the device.

### SUMMARY OF THE INVENTION

The present invention provides a single sac ventricular assist device having a bearingless electromagnetic drive for assisting or replacing the function of one or both ventricles of the heart.

The invention is defined by the appended claims.

Broadly stated, the present invention provides a ventricular assist device for pumping blood between an inlet and an outlet, said device comprising a frame; a compressible chamber disposed within said frame and connected between said inlet and said outlet; a first one-way valve for providing fluid communication from said inlet to said chamber; a second one-way valve for providing fluid communication from said chamber to said outlet, and a bearingless electromagnetic drive unit disposed within said frame including one or more coils wound about one or more cores and an armature disposed between said cores, said armature having one or more magnets each having a pair of magnetic poles, wherein said bearingless electromagnetic drive unit, when energized, providing a force on said armature towards said compressible chamber during an eject stroke wherein fluid is expelled from said compressible chamber to said outlet.

In accordance with one aspect of the present invention, a ventricular assist device has the advantage of providing a single sac configuration that eliminates a pump and corresponding transfer conduit and valve housing associated with having two pumps in known dual pump devices. The ventricular assist device according to the present invention has an inlet and an outlet and has the advantage of utilizing the corresponding valves and conduits of a known system as shown in FIG. 1 for the inlet and outlet.

The ventricular assist device according to the present invention has a further advantage of requiring simpler implant electronics and control. A related advantage of the ventricular assist device according to the present invention is that it preferably provides slower ejection that allows use of a smaller vent tube so as to reduce skin penetration and the associated infection risk.

The ventricular assist device according to the present invention has a further advantage in that blood transits only one pump such that the pressure drop is halved. The left ventricular pressure (LVP) does not contribute to the outflow pressure.

These and other embodiments, features, aspects, and advantages of the invention will become better understood with regard to the following description, appended claims, and accompanying drawings.

### BRIEF DESCRIPTION OF THE DRAWINGS

The foregoing aspects and the attendant advantages of the present invention will become more readily appreciated by reference to the following detailed description, when taken in conjunction with the accompanying drawings, wherein:
FIG. 1 is perspective view of a blood pump shown in U.S. Patent No. 6,264,601 in which variable-volume chambers operate in series;
FIGs. 2A to 2F are schematic views of the blood pump in FIG. 1, illustrating the variable-volume chambers of the pump operating in series;
FIG. 3 is a schematic view of a single sac bearingless pump drive unit (PDU) of a VAD according to an embodiment of the present invention;
FIG. 4 is a schematic illustrating exemplary operation of the PDU in FIG. 3 of a VAD at the start of the eject stroke;
FIG. 5 is a schematic illustrating exemplary operation of the PDU in FIG. 3 of a VAD at the end of the eject stroke;
FIG. 6 is a schematic illustrating exemplary operation of the PDU in FIG. 3 of a VAD at the "ready to fill" stage;
FIG. 7 is a schematic illustrating the start of the eject stroke for an PDU of a VAD including an energy storage element according to a preferred embodiment of the present invention;
FIG. 8 is a schematic illustrating the end of the eject stroke for the PDU in FIG. 7;
FIG. 9 is a schematic illustrating the "ready to fill" stage for the PDU in FIG. 7, with the armature retracted;
FIG. 10 is a side view illustrating the armature support spring with the armature retracted for a single sac PDU of a VAD according to one aspect of an embodiment of the present invention;
FIG. 11 is a side view illustrating the armature support spring in FIG. 10 with the pump ejected according to one aspect of an embodiment of the present invention;
FIG. 12 is a top view illustrating an armature support spring in FIG. 10;
FIG. 13 is a side view illustrating an embodiment of the pump stabilization flexure for a single sac PDU of a VAD, with the chamber filled, according to an alternative embodiment of the present invention.
FIG. 14 is a side view illustrating the pump stabilization flexure of FIG. 13 after ejection of the contents of the chamber;
FIG. 15 is a top view illustrating the pump stabilization flexure of FIG. 13;
FIG. 16 illustrates a cross section of a PDU and an inlet of a VAD having an armature decoupled from the drive unit and a chamber located between the armature and a coil-less eject stator core according to an alternate embodiment of the present invention;
FIG. 17 illustrates the end of the eject stroke for the PDU in FIG. 16;
FIG. 18 illustrates the armature retracted and the start of pump filling for the PDU in FIG. 16;
FIG. 19 illustrates a sectional view through the permanent magnet of the PDU 600 in FIG. 16 with the armature position reversed;
FIG. 20 illustrates a center section view of the PDU in FIG. 19;
FIG. 21 illustrates a sectional view through the permanent magnet of an embodiment of the PDU in FIG. 16 with coils wound on respective legs of one core and the armature position reversed from that in FIG. 16; and
FIG. 22 illustrates a pump center section of the PDU in FIG. 21.

Reference symbols are used in the Figures to indicate certain components, aspects or features shown therein, with reference symbols common to more than one Figure indicating like components, aspects or features shown therein.

### DETAILED DESCRIPTION OF THE INVENTION

The ventricular assist device according to the present invention is for pumping blood between an inlet and an outlet. The bearingless electromagnetic drive unit of the VAD is used to drive a single blood pump. According to a preferred embodiment of the present invention, the drive unit actively forces an armature toward a compressible chamber during an ejection stroke, but decouples the armature from the drive unit in a retraction stroke. Following retraction, the compressible chamber is passively filled with blood from the inlet.

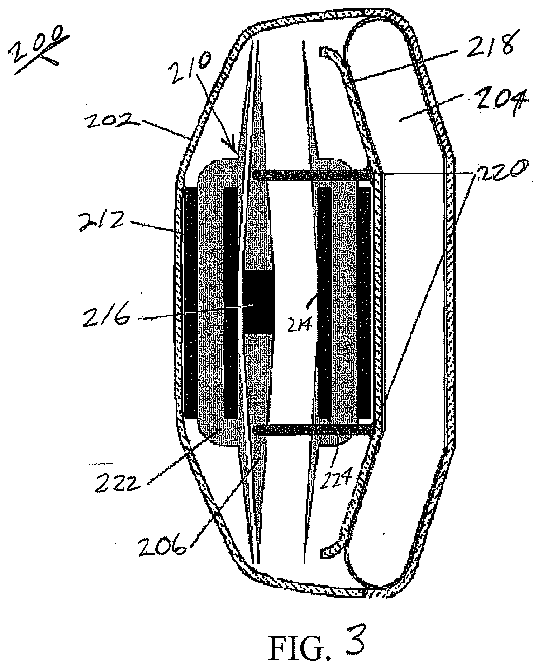

FIG. 3 is a schematic view of a single sac bearingless pump drive unit (PDU) 200 of a VAD according to an embodiment of the present invention. As seen in FIG. 3, PDU 200 includes a frame 202, a compressible chamber 204, a bearingless electromagnetic drive unit 210, and an armature 206. The compressible chamber 204 is preferably a flexible sac. The compressible chamber 204 is disposed within the frame 202 and connected between an inlet and an outlet (not shown in FIG. 3). The ventricular assist device according to the present invention has the advantage of enabling utilization of known inlet and outlet arrangements, e.g., as describe above and shown in FIG. 1. For convenience, details of the inlet and outlet arrangement are not shown in FIGs. 3-22, except where indicated. The VAD of the present invention preferably includes a one-way valve for providing fluid communication from the inlet to the chamber and a second one-way valve for providing fluid communication from the chamber to the outlet. The bearingless electromagnetic drive unit 210 is disposed within the frame 202 and includes a coil 212 wound about a core 222 and a coil 214 wound about a core 224. The armature 206 is disposed between the compressible chamber 204 and the frame 202. The armature 206 includes a magnet 216. Magnet 216 is preferably a permanent magnet having a pair of magnetic poles.

The bearingless electromagnetic drive unit 210, when energized, providing a force on the armature 206 towards the compressible chamber 204 during an eject stroke wherein fluid is expelled from the compressible chamber 204 to an outlet (not shown in FIG. 3). The coils 212, 214 are positioned so as to generate a coil flux path through a magnetically permeable portion of the armature 206 which displaces the armature 206 toward the compressible chamber 204. The armature 206 is unstable in a central position between the drive unit 210 and the compressible chamber 204 and is biased away from the chamber 204 when the coils 212, 214 are not energized.

FIG. 3 shows a movable plate identified as pusher plate 218. Pusher plate may be attached to compressible chamber 204. Alternatively, the movable plate is part of the armature 206. The movement of armature 206 causes pusher plate 218 and push rods 220 to compress chamber 204 during the eject stroke. It should be appreciated that the force provided on the armature 206 may be exerted on the chamber 204 by other coupling to the chamber 204 included for the armature 206.

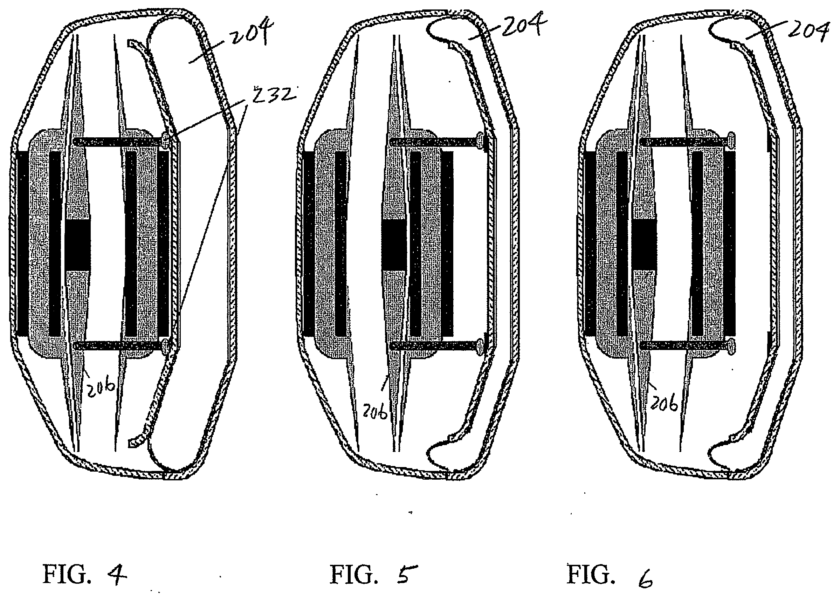

FIG. 4 is a schematic illustrating exemplary operation of the PDU 200 in FIG. 3 of a VAD at the start of the eject stroke. FIG. 4 shows pads 232 alternatively coupled to the push rod 220. In the "start of eject" stage shown in FIG. 4, the armature 206 is in a retracted position with the pump filled. The armature 206 is driven to the right, ejecting blood from pump.

FIG. 5 is a schematic illustrating exemplary operation of the PDU 200 in FIG. 3 of a VAD at the end of the eject stroke. As seen in FIG. 5, compressible chamber 204, i.e. the preferred single sac, is in the compressed state due to expelling of fluid therefrom to an outlet (not shown in FIG. 5). Preferably, a movable plate is attached to the compressible chamber 204. FIG. 5 shows an exemplary movable plate, pusher plate 218. Alternatively, the pusher plate 218 is not attached to the compressible chamber 204. According to another alternative, the movable plate is part of the armature 206. The present invention is not limited to the pusher plate 218 as shown in FIG. 5; other means may alternatively be used.

In operation, the armature 206 is decoupled from the compressible chamber 204, and the pusher plate attached thereto, after completion of the eject stroke, shown in FIG. 5, such that the armature 206 retracts in a retraction stroke. Following the retraction stroke, the compressible chamber 204 is ready to fill and is then passively filled with blood from an inlet. FIG. 6 is a schematic illustrating exemplary operation of the PDU in FIG. 3 of a VAD at the "ready to fill" stage.

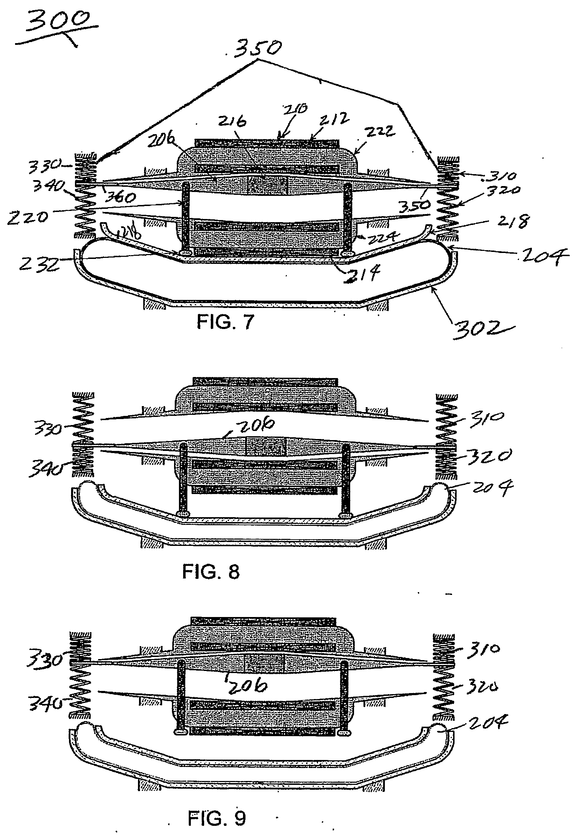

The VAD according to the present invention preferably includes an energy storage element (for convenience, not shown in FIGs. 3-6) adapted to store and release energy from the bearingless electromagnetic drive unit. FIG. 7 is a schematic illustrating the start of the eject stroke for an PDU 300 of a VAD including an energy storage element 350 according to a preferred embodiment of the present invention. FIG. 7 also shows schematically anchoring of the cores 222, 224, along with additional housing and frame portions 302.

One or more springs are preferably positioned between the frame and the armature of the VAD to exert a spring force on the armature. Energy is stored in said energy storage element 350 during the retraction stroke and is delivered during the eject stroke of the VAD of the present invention. The energy storage element 350 in FIG. 7 includes a spring 310 and a spring 320, each coupled to opposing sides of a radial arm 350. The energy storage element 350 also includes a spring 330 and a spring 340, each coupled to opposing sides of a radial arm 360.

The springs 310, 320, 330, 340 are preferably helical compression springs arranged in pairs, as shown in FIG. 7. For simplicity, the springs 310, 320, 330, 340 are shown installed symmetrically with respect to the mid-point of the stroke of the armature 206, via corresponding radials arms 350 and 360. The anchor points of the springs 310, 320, 330, 340 are offset some distance towards the eject position according to a preferred embodiment of the present invention, which provides a constant bias force in the eject direction.

According to the preferred embodiment shown in FIG, 7, the springs 310, 320,330,340 exert a spring force on the armature 206. The magnet 216 generates a magnet force on the armature 206 resulting from the attraction of magnet 216 to the frame 302 when the coils 212, 214 are not electrically energized. The energized coils 212,214 generates a coil force on the armature 206 that is approximately independent of the position of the armature 206 and that varies according to the degree of energization of the coils 212, 214. Preferably, the sum of the spring force, the magnet force, and the coil force is approximately independent of the position of the armature 206, and varies according to the degree of energization of the coils 212, 214.

Energy stored in the springs during armature retraction is delivered to the pump during the eject stroke. FIG. 8 is a schematic illustrating the end of the eject stroke for the PDU 300 in FIG. 7. As seen in FIG. 8, the spring 310 and 330 which were in a compressed state in FIG. 7 are expanded as shown in FIG. 8 for ejection, with the opposite occurring for springs 320 and 340. The armature 206 is decoupled from the compressible chamber 204 after completion of the eject stroke such that armature 206 retracts in a retraction stroke. Following the retraction stroke, the compressible chamber 204 is passively filled with blood from an inlet. FIG. 9 is a schematic illustrating the "ready to fill" stage for the PDU 300 in FIG. 7, with the armature 206 retracted.

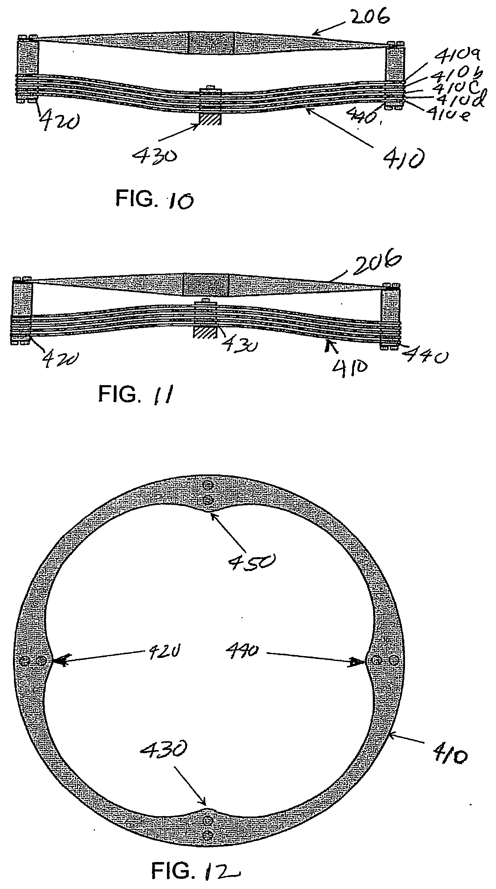

FIGs. 10-12 show an alternate spring arrangement for a single sac PDU according to an embodiment of the present invention. The coil springs shown in FIG. 7 are replaced with a circular leaf spring in the alternate embodiment shown in FIGs. 10-12 in order to provide a more compact overall assembly. FIG. 10 is a side view illustrating the armature support spring 410 with the armature retracted for a single sac PDU of a VAD according to one aspect of an embodiment of the present invention; FIG. 11 is a side view illustrating the armature support spring 410 in FIG. 10 with the pump ejected according to one aspect of an embodiment of the present invention. The spring is preferably a flat ring comprised of two or more rings arranged in layers. As shown in FIGs. 10 and 11, spring 410 has layers 410a, 410b, 410c, 410d, and 410e. The layers 410a, 410b, 410c, 410d, 410e are attached to at least two movable portions 420, 440 of the VAD and to at least two stationary portions 430, 450 thereof, wherein the layers are coupled together at the attachments 420, 430, 440. FIG. 12 is a top view illustrating an armature support spring in Fig. 10.

The springs shown in FIGs. 10-12 are illustrated as symmetrical about the mid-stroke, but in practice the fixed anchor points, 430, 450 would be offset to create a bias force towards ejection. The number of layers in the spring depends on the thickness of the spring material and the desired spring constant K. As shown in FIGs. 10 and 11, the individual springs are spaced apart to prevent rubbing between adjacent springs. The resultant relatively thick stack height also provides the benefit of increased resistance to tilting, as required to stabilize the armature 206.

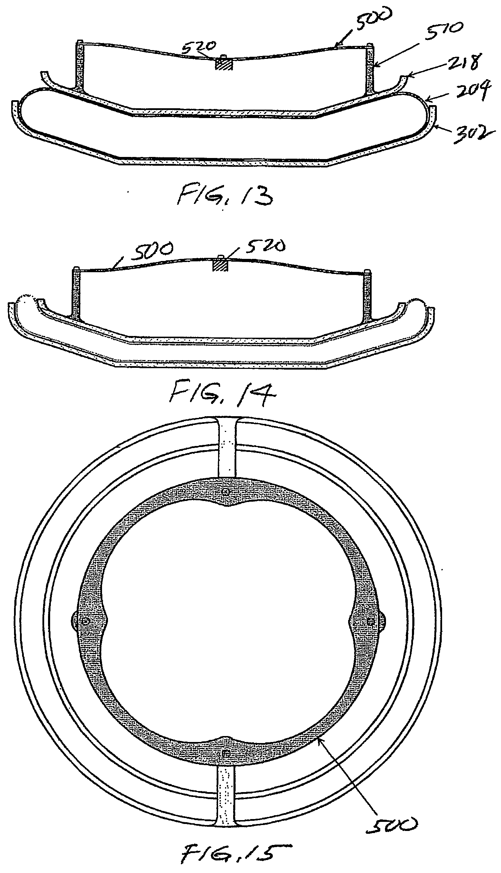

FIGs. 13-15 illustrate schematically a pump stabilization flexure aspect for pusher plate stabilization according to an alternative embodiment of the present invention. FIG. 13 is a side view illustrating an embodiment of the pump stabilization flexure 500 for a single sac PDU of a VAD, with the chamber filled, according to an alternative embodiment of the present invention. FIG. 14 is a side view illustrating the pump stabilization flexure of FIG. 13 after ejection of the contents of the chamber. The sac 204 shown in FIGs. 13-15 resists translation of the pusher plate 218 normal to its axis of travel, but provides little resistance to tilt. The stabilization flexure 500 attaches to the pusher plate at a plane offset from that of the sac, and resists translation of the pusher plate in that plane. The resulting lateral constraint provided in two offset planes translates into tilt resistance. FIG. 15 is a top view illustrating the pump stabilization flexure of FIG. 13.

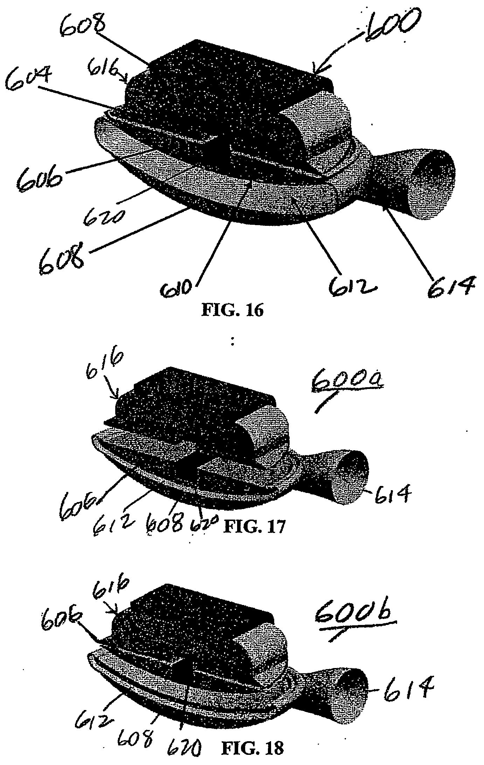

FIG. 16 illustrates a cross section of a PDU 600 of a VAD having an armature decoupled from the drive unit and a chamber located between the armature and a coil-less eject stator core according to an alternate embodiment of the present invention. The PDU 600 includes a drive unit 616, an armature 606, and a compressible chamber 612. The PDU includes an inlet and an outlet. An exemplary inlet 614 is shown in FIG. 16, the corresponding outlet (not shown in FIG. 16) preferably has the same structure. The armature has two tapered ends and a permanent magnet 620 therebetween. The compressible chamber 612 is preferably a flexible sac. The drive unit 616 comprises a single coil 602 wound around the center section of a U-shaped core 604, and another core 608. The core 608 is coil-less. A separate movable plate 610 is alternatively included. The movable plate 610 may be attached to the compressible chamber 612. The armature 606 may be decoupled from the movable plate 610. FIG. 16 also illustrates the start of the eject stroke, with the chamber 612 in a filled state.

FIG. 17 illustrates the end of the eject stroke for the PDU 600 in FIG. 16. The end of the eject stroke stage shown in FIG. 17 is identified as 600a. FIG. 18 illustrates the armature retracted and the start of pump filling for the PDU 600 in FIG. 16. The stage shown in FIG. 18 is identified as 600b. The armature 606 in FIG. 18 is decoupled from the movable plate 610, allowing passive filling. The chamber 612 in PDU 600 is located within one of the two magnetic gaps, i.e., between the armature 606 and the eject core 608, in a kind of integrated configuration. The PDU 600a has coil 602 wound around only one of the two cores, the ejector core 608 has no coil. The coil-less eject core 608 provides the manufacturing advantage in that it eliminates the need to run wires to a coil on the far side of the PDU.

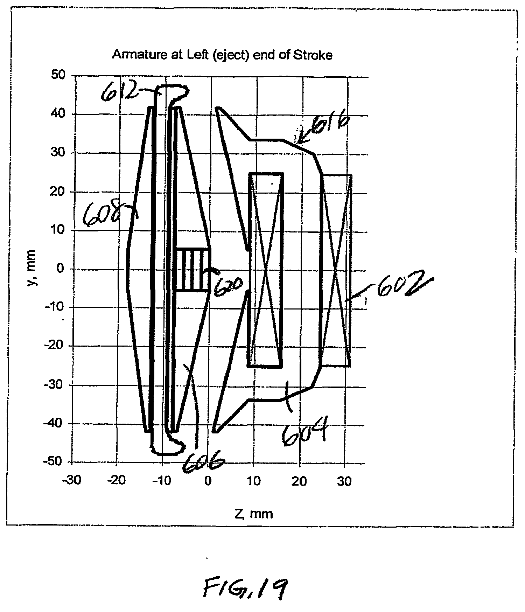

FIGs. 19-22 are view of embodiments of the PDU in FIGs. 16-18 with the position of armature 600 reversed. That is, in FIGs. 16-18, the flat side of the armature 606 is positioned towards core 604 (also identified as the retract stator), and the domed, angled side of the armature 606 is towards the chamber 612. The armature 606 is positioned the opposite way in FIGs. 19-22, i.e., with the flat side toward the chamber 612 and the domed, angled side toward core 604, the retract stator. This s structure and arrangement of the armature 606 in FIGs. 16-18 provides two advantages relative to the reverse shown for the armature 606 in FIGs. 19-22. Positioning the flat poles of armature 606 towards the retract stator, which closes to a relatively small gap, minimizes tilt forces due to lateral misalignment. Positioning the domed, angled side of armature 606 towards the chamber 612 yields a more rigid pusher plate 610, and the corresponding domed housing shown in FIGs. 16-18 provides a more anatomic "oyster" overall package shape for improved implantability.

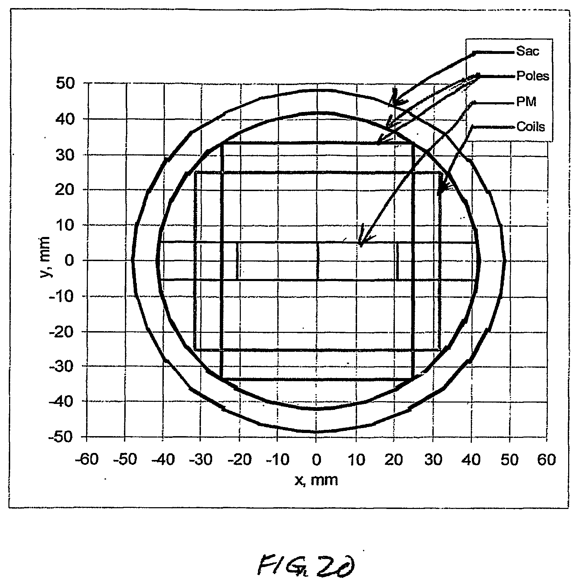

FIG. 19 illustrates a sectional view through the permanent magnet of the PDU 600 in FIG. 16 with the armature position reversed. FIG. 20 illustrates a center section view of the PDU in FIG. 19. For the view in FIG. 20, the cores and armature, less the PM portion, are identified as "poles". The inlet 614 is not shown in FIGs. 19 and 20. Alternatively, there are coils on both cores of the integrated, decoupled PDU 600.

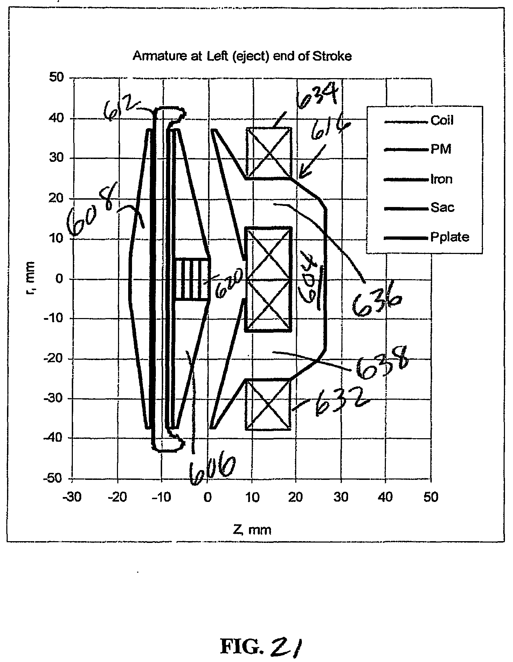

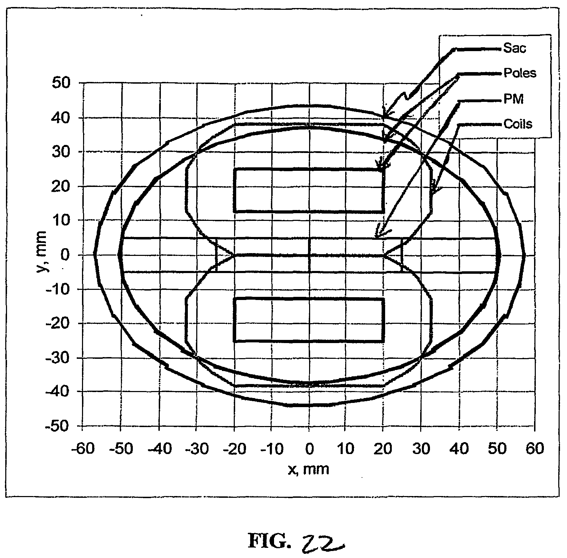

According to another alternative embodiment, a pair of coils is wound around one of the cores, and the other core is coil-less, as shown in FIG. 21. FIG. 21 illustrates a sectional view through the permanent magnet of an embodiment of the PDU 600 in FIG. 16 with coils 632, 634 wound on respective legs 638, 636 of the core 604 and the armature position reversed. FIG. 22 illustrates a pump center section of the PDU in FIG. 21. For the view in FIG. 22, the cores and armature, less the PM portion, are identified as "poles". Alternatively, there are pairs of coils on both cores of the integrated, decoupled PDU 600.

Preferably, there is full-to-empty ("automatic") ejection of the sac. Although a partial fill is possible, it is not energetically efficient. For a partial fill, slack armature motion must be retarded to avoid impact. A partial eject is possible according to an embodiment of the single sac VAD of the present invention. For a partial eject, eject current is terminated if the pump "stalls" on the peak of the aortic pressure.

The single sac VAD according to the present invention provides for controlled pressure resulting in lowered inlet and outlet valve stress. At full fill, initial eject pressure for an embodiment of the single sac VAD of the present invention is solely from coil current force, there is an inherent ramp up due to self-inductance. As a result, inlet inflow valve stress is simple to control for the present invention.

The single sac VAD according to the present invention has the advantage of providing a major size reduction by eliminating one pump, saving approx. 180 ml in one example, and eliminating the transfer conduit and valve housing. The single sac VAD according to the present invention has the advantage of reducing pressure requirements since the blood transits only one pump, halving the pressure drop. The left ventricular pressure does not contribute to outflow pressure.

In an exemplary single sac VAD according to the present invention, the DMD retracts quickly, e.g., 50 msec, saving 75% dissipated energy vs. 200 msec pump stroke. (For 60 ml stroke at 300 ml/sec nominal flow). In one example, 37% of the full cycle was dissipated energy is saved. Peak power is the eject power. The use of passive filling results in simpler electronics and control circuitry.

According to an embodiment, the device of the present invention operates with use of a percutaneous tube vented to the atmosphere for enabling passive filling of the chamber. The single sac VAD according to a preferred embodiment of the present invention provides a slower ejection that enables use of a smaller vent tube: 5 mm lumen (5 mmHg ΔP in ½ m length with 300 ml/sec flow). According to another embodiment, the device of the present invention operates with use of an implanted volume displacement chamber for enabling passive filling of the chamber without venting to the atmosphere.

## Claims

1. A ventricular assist device for pumping blood between an inlet (614) and an outlet, comprising:
a frame (202, 302);
a compressible chamber (204, 612) disposed within said frame (202, 302) and connected between said inlet (614) and said outlet;
a first one-way valve for providing fluid communication from said inlet (614) to said chamber (204, 612);
a second one-way valve for providing fluid communication from said chamber (204, 612) to said outlet,
**characterized in that**
said chamber (204, 612) is a single compressible chamber disposed within and adjacent to a first lateral side of said frame (202, 302)
and that a bearingless electromagnetic drive unit (210, 300, 616) is disposed within and adjacent to a second lateral side of said frame (202, 302), comprising two cores (222, 224, 604,608), one or more coils (212, 214, 602, 632, 634) wound about one or both of said cores, and an armature (206, 606, 600) disposed between said cores, said armature (206, 606, 600) having one or more magnets (216, 620) each having a pair of magnetic poles, wherein said bearingless electromagnetic drive unit (210, 300, 616), when energized, providing a force on said armature (206, 606, 600) towards said compressible chamber (204, 612) during an eject stroke wherein fluid is expelled from said compressible chamber (604, 612) to said outlet.

2. The ventricular assist device of claim 1, further comprising:
an energy storage element (350,) adapted to store and release energy from said bearingless electromagnetic drive unit (210, 300, 616);
wherein, during the eject stroke, electric power delivered to said bearingless electromagnetic drive unit (210, 300, 616) and energy stored in said storage element (350) are delivered to said armature (206, 606, 600) for forcing said armature toward said compressible chamber (204,612)
wherein said armature (206, 606, 600) is decoupled from said compressible chamber (204 612); after completion of said eject stroke such that said armature (206, 606, 600) retracts in a retraction stroke; and wherein, following said retraction stroke, said compressible chamber (204, 612) is passively filled with blood from said inlet (614) and energy stored in said storage element (350) during said retraction stroke is delivered during said eject stroke.

3. The ventricular assist device of claim 2,
wherein said storage element (350) includes one or more springs (310, 320, 330, 340) positioned between said frame (202, 302); and said armature (206, 606, 600) to exert a spring force on said armature (206, 606, 600);
wherein said one or more magnets (216, 620) generate a magnet force on said armature (206, 606, 600) resulting electrically energized and
wherein said one or more energized coils (212, 214, 602, 632, 634) generates a coil force on said armature (206, 606, 600) that is approximately independent of the position of said armature (206, 606, 600) and that varies according to the degree of energization of said one or more coils (212, 214, 602, 632, 634).

4. The ventricular assist device of claim 3, wherein the sum of said spring force, said magnet force, and said coil force is approximately independent of the position of said armature (206, 606, 600), and varies according to the degree of energization of said one or more coils (212, 214, 602, 632, 634).

5. The ventricular assist device of claim 3, wherein said spring is a flat ring.

6. The ventricular assist device of claim 5, wherein said flat ring is comprised of two or more rings arranged in layers (410a, 410b, 410c, 410d, 410e).

7. The ventricular assist device of claim 6, wherein said layers (410a, 410b, 410c, 410d, 410e) are attached to at least two movable portions (420, 440) of said device and to at least two stationary portions (430, 450) of said device, wherein said layers (410a, 410b, 410c, 410d, 410e) are coupled together at the attachments (420, 430, 440).

8. The ventricular assist device of claim 3, wherein said spring comprises one or more coil springs.

9. The ventricular assist device of claim 1, further comprising a percutaneous tube vented to the atmosphere for enabling passive filling of said chamber (204, 612).

10. The ventricular assist device of claim 1, further comprising an implanted volume displacement chamber for enabling passive filling of said chamber without venting to the atmosphere.

11. The ventricular assist device of claim 1, wherein said outlet is adapted for coupling to an aorta and wherein said inlet (614) is adapted for coupling to a ventricle to enable passive filling of said chamber (204, 612) from said ventricle.

12. The ventricular assist device of claim 1, wherein said compressible chamber (204, 612) is a flexible sac.

13. The ventricular assist device of claim 12, wherein a movable plate (218, 610) is attached to said flexible sac.

14. The ventricular assist device of claim 1, wherein said armature (206, 606, 600) further includes a movable plate (218, 610).

15. The ventricular assist device of claim 1, wherein said one or more coils (212, 214, 602, 632, 634) are positioned so as to generate a coil flux path through magnetically permeable portion of the armature (206, 606, 600), and wherein the coil flux generated by said one or more coils (212, 214, 602, 632, 634) displaces said armature (206, 606, 600) toward said compressible chamber (204, 612).

16. The ventricular assist device of claim 1, wherein said armature (206, 606, 600) is unstable in a central position between said drive unit (210, 300, 616) and said chamber (204, 612); and wherein said armature (206, 606, 600) is biased away from said chamber (204, 612) when said coils (212, 214, 602, 632, 634) are not energized.

17. The ventricular assist device of claim 1, wherein a first one of said cores (222, 224, 604, 608) is coil-less, and one or more of said coils (212, 214, 602, 632, 634) are wound around a second one of said cores (222, 224, 604, 608).

18. The ventricular assist device of claim 17, wherein a pair of said coils (212, 214, 602, 632, 634) is wound around the second one of said cores (222, 224, 604, 608).

19. The ventricular assist device of claim 18, wherein another pair of said coils (212, 214, 602, 632, 634) is wrapped around said first one of said cores (222, 224, 604, 608).

20. The ventricular assist device of claim 17, wherein one said coil (212, 214, 602, 632, 634) is wound around the second one of said cores (222, 224, 604, 608).

21. The ventricular assist device of claim 20, wherein another said coil (212, 214, 602, 632, 634) is wrapped around the first one of said cores (222, 224, 604, 608).

22. The ventricular assist device of claim 17, wherein said chamber (204, 612) is positioned between said armature (206, 606, 600) and said coil-less core (222, 224, 604, 608) and wherein said armature (206, 606, 600) retracts toward said second one of said cores core (222, 224, 604, 608) during said retraction stroke.

23. The ventricular assist device of claim 1, wherein one or more of said coils (212, 214, 602, 632, 634) are wound around a first one of said cores (222, 224, 604, 608), and one or more of said coils (212, 214, 602, 632, 634) are wound around a second one of said cores (222, 224, 604, 608), and wherein said chamber (204, 612) is positioned between said armature (206, 606, 600) and said first one of said cores (222, 224, 604, 608) and wherein said armature (206, 606, 600) retracts toward said second one of said cores (222, 224, 604, 608) during said retraction stroke.

## Patentansprüche

1. Ventrikuläre Unterstützungsvorrichtung zum Pumpen von Blut zwischen einem Einlass (614) und einem Auslass, umfassend:
ein Gehäuse (202, 302);
eine innerhalb dieses Gehäuses (202, 302) angeordnete und zwischen dem Einlass (614) und dem Auslass angeschlossene komprimierbare Kammer (204, 612),
ein erstes Rückschlagventil zur Bereitstellung einer Fluidverbindung vom Einlass (614) zur Kammer (204, 612);
ein zweites Rückschlagventil zur Bereitstellung einer Fluidverbindung von der Kammer (204, 612) zum Auslass,
**dadurch gekennzeichnet, dass**
die Kammer (204, 612) eine einzige komprimierbare Kammer ist, die innerhalb und an eine erste laterale Seite des Rahmens (202, 302) angrenzend angeordnet ist,
und dass eine lagerlose elektromagnetische Antriebseinheit (210, 300, 616) innerhalb und an eine zweite laterale Seite des Rahmens (202, 302) angrenzend angeordnet ist, umfassend zwei Kerne (222, 224, 604, 608), eine oder mehrere Spulen (212, 214, 602, 632, 634), die um einen oder beide Kerne gewickelt sind, und einen Anker (206, 606, 600), der zwischen den Kernen angeordnet ist und
einen oder mehrere Magneten (216, 620) aufweist, die jeweils ein Paar magnetische Pole aufweisen, wobei die lagerlose elektromagnetische Antriebseinheit (210, 300, 616), wenn sie mit Strom versorgt wird, während eines Ausstoßtakts, eine Kraft auf den Anker (206, 606, 600) in Richtung der komprimierbaren Kammer (204, 612) ausübt, wodurch Flüssigkeit aus der komprimierbaren Kammer (604, 612) zum Auslass ausgestoßen wird.

2. Ventrikuläre Unterstützungsvorrichtung nach Anspruch 1, weiterhin umfassend:
ein Energiespeicherelement (350), das dazu eingerichtet ist, Energie von der lagerlosen elektromagnetischen Antriebseinheit (210, 300, 616) zu speichern und auszugeben;
wobei während des Ausstoßtakts, ein elektrischer Strom, der der lagerlosen elektromagnetischen Antriebseinheit (210, 300, 616) zugeführt wird, sowie eine im Speicherelement (350) gespeicherte Energie, dem Anker (206, 606, 600) zugeführt werden, um den Anker in Richtung der komprimierbaren Kammer (204, 612) zu treiben,
wobei der Anker (206, 606, 600) nach Abschluss des Ausstoßtakts derart von der komprimierbaren Kammer (204, 612) entkoppelt wird, dass der Anker (206, 606, 600) während eines Rückstellungstakts zurückgestellt wird; und wobei, nach dem Rückstellungstakt, die komprimierbare Kammer (204, 612) passiv mit Blut vom Einlass (614) gefüllt wird und Energie, die während des Rückstellungstakts im Speicherelement (350) gespeichert wurde, während des Ausstoßtakts zugeführt wird.

3. Ventrikuläre Unterstützungsvorrichtung nach Anspruch 2, wobei das Speicherelement (350) eine oder mehrere Federn (310, 320, 330, 340) umfasst, die zwischen dem Gehäuse (202, 302) und dem Anker (206, 606, 600) platziert sind, um eine Federkraft auf den Anker (206, 606, 600) auszuüben; wobei das eine oder die mehreren Magnete (216, 620) eine Magnetkraft auf den Anker (206, 606, 600) ausüben, wodurch dieser elektrisch versorgt wird, und wobei die eine oder mehreren Spulen (212, 214, 602, 632, 634) auf den Anker (206, 606, 600) eine Spulenkraft ausüben, die ungefähr unabhängig von der Position des Ankers (206, 606, 600) ist und je nach Bestromungsgrad der einen oder mehreren Spulen (212, 214, 602, 632, 634) variiert.

4. Ventrikuläre Unterstützungsvorrichtung nach Anspruch 3, wobei die Summe der Federkraft, der Magnetkraft und der Spulenkraft ungefähr unabhängig von der Position des Ankers (206, 606, 600) ist und je nach Bestromungsgrad der einen oder mehreren Spulen (212, 214, 602, 632, 634) variiert.

5. Ventrikuläre Unterstützungsvorrichtung nach Anspruch 3, wobei die Feder ein flacher Ring ist.

6. Ventrikuläre Unterstützungsvorrichtung nach Anspruch 5, wobei der flache Ring aus zwei oder mehreren in Schichten (440a, 410b, 410c, 410d, 410e) angeordneten Ringen besteht.

7. Ventrikuläre Unterstützungsvorrichtung nach Anspruch 6, wobei die Schichten (410a, 410b, 410c, 410d, 410e) mit mindestens zwei beweglichen Teilen (420, 440) der Vorrichtung und mit mindestens zwei ortsfesten Teilen (430, 450) der Vorrichtung verbunden sind, wobei die Schichten (410a, 410b, 410c, 410d, 410e) an den Verbindungen (420, 430, 440) miteinander gekoppelt sind.

8. Ventrikuläre Unterstützungsvorrichtung nach Anspruch 3, wobei die Feder eine oder mehrere Spiralfedern umfasst.

9. Ventrikuläre Unterstützungsvorrichtung nach Anspruch 1, weiter umfassend eine perkutane Sonde, die zur Atmosphäre hin abgeleitet wird, um eine passive Füllung der Kammer (204, 612) zu ermöglichen.

10. Ventrikuläre Unterstützungsvorrichtung nach Anspruch 1, weiter umfassend eine implantierte Volumenverlagerungskammer, um eine passive Füllung der Kammer ohne Ableitung zur Atmosphäre zu ermöglichen.

11. Ventrikuläre Unterstützungsvorrichtung nach Anspruch 1, wobei der Auslass zur Verkopplung mit einer Aorta geeignet ist, und wobei der Einlass (614) zur Verkopplung mit einem Ventrikel geeignet ist, um eine passive Füllung der Kammer (204, 612) aus dem Ventrikel zu ermöglichen.

12. Ventrikuläre Unterstützungsvorrichtung nach Anspruch 1, wobei die komprimierbare Kammer (204, 612) ein flexibler Beutel ist.

13. Ventrikuläre Unterstützungsvorrichtung nach Anspruch 12, wobei eine bewegliche Platte (218, 610) am flexiblen Beutel befestigt ist.

14. Ventrikuläre Unterstützungsvorrichtung nach Anspruch 1, wobei der Anker (206, 606, 600) außerdem eine bewegliche Platte (218, 610) umfasst.

15. Ventrikuläre Unterstützungsvorrichtung nach Anspruch 1, wobei die eine oder mehreren Spulen (212, 214, 602, 632, 634) derart positioniert sind, das sie einen Spulenflussweg durch magnetisch durchlässige Teile des Ankers (206, 606, 600) erzeugen, und wobei der durch die eine oder mehreren Spulen (212, 214, 602, 632, 634) erzeugte Spulenfluss den Anker (206, 606, 600) in Richtung der komprimierbaren Kammer (204, 612) verdrängt.

16. Ventrikuläre Unterstützungsvorrichtung nach Anspruch 1, wobei der Anker (206, 606, 600) in einer zentralen Stellung zwischen der Antriebseinheit (210, 300, 616) und der Kammer (204, 612) unstabil ist und wobei der Anker (206, 606, 600) von der Kammer (204, 612) weg ausgerichtet ist, wenn die Spulen (212, 214, 602, 632, 634) nicht mit Strom versorgt werden.

17. Ventrikuläre Unterstützungsvorrichtung nach Anspruch 1, wobei ein erster der Kerne (222, 224, 604, 608) spulenlos ist und einer oder mehrere der Spulen (212, 214, 602, 632, 634) um einen zweiten der Kerne (222, 224, 604, 608) gewickelt sind.

18. Ventrikuläre Unterstützungsvorrichtung nach Anspruch 17, wobei ein Paar der Spulen (212, 214, 602, 632, 634) um den zweiten der Kerne (222, 224, 604, 608) gewickelt ist.

19. Ventrikuläre Unterstützungsvorrichtung nach Anspruch 18, wobei ein anderes Paar der Spulen (212, 214, 602, 632, 634) um den ersten der Kerne (222, 224, 604, 608) gewickelt ist.

20. Ventrikuläre Unterstützungsvorrichtung nach Anspruch 17, wobei eine der Spulen (212, 214, 602, 632, 634) um den zweiten der Kerne (222, 224, 604, 608) gewickelt ist.

21. Ventrikuläre Unterstützungsvorrichtung nach Anspruch 20, wobei eine andere der Spulen (212, 214, 602, 632, 634) um den ersten der Kerne (222, 224, 604, 608) gewickelt ist.

22. Ventrikuläre Unterstützungsvorrichtung nach Anspruch 17, wobei die Kammer (204, 612) zwischen dem Anker (206, 606, 600) und dem spulenlosen Kern (222, 224, 604, 608) positioniert ist, und wobei sich der Anker (206, 606, 600) während des Rückstellungstakts in Richtung des zweiten der Kerne (222, 224, 604, 608) zurückzieht.

23. Ventrikuläre Unterstützungsvorrichtung nach Anspruch 1, wobei eine oder mehrere der Spulen (212, 214, 602, 632, 634) um einen ersten der Kerne (222, 224, 604, 608) gewickelt sind, und eine oder mehrere der Spulen (212, 214, 602, 632, 634) um einen zweiten der Kerne (222, 224, 604, 608) gewickelt sind, und wobei die Kammer (204, 612) zwischen dem Anker (206, 606, 600) und dem ersten der Kerne (222, 224, 604, 608) positioniert ist und wobei sich der Anker (206, 606, 600) während des Rückstellungstakts in Richtung des zweiten der Kerne (222, 224, 604, 608) zurückzieht.

## Revendications

1. Dispositif d'assistance ventriculaire pour pomper le sang entre une entrée (614) et une sortie, comportant :
un cadre (202, 302) ;
une chambre compressible (204, 612) disposée à l'intérieur dudit cadre (202, 302) et connectée entre ladite entrée (614) et ladite sortie ;
une première valve anti-retour destinée à assurer une communication fluidique entre ladite entrée (614) et ladite chambre (204, 612) ;
une seconde valve anti-retour destinée à assurer une communication fluidique entre ladite chambre (204, 612) et ladite sortie,
**caractérisé en ce que**
ladite chambre (204, 612) est une unique chambre compressible disposée à l'intérieur de et adjacente à un premier côté latéral dudit cadre (202, 302) et qu'une unité de commande électromagnétique sans palier (210, 300, 612) est disposée à l'intérieur de et adjacente à un second côté latéral dudit cadre (202, 302), comportant deux noyaux (222, 224, 604, 608), une ou plusieurs bobines (212, 214, 602, 632, 634) enroulées autour d'un ou plusieurs desdits noyaux, et une armature (206, 606, 600) disposée entre lesdits noyaux, ladite armature (206, 606, 600) comportant un ou plusieurs aimants (216, 620) ayant chacun une paire de pôles magnétiques, où, lorsqu'elle est alimentée, ladite unité de commande électromagnétique sans palier (210, 300, 616) délivre une force sur ladite armature (206, 606, 600) en direction de ladite chambre compressible (204, 612) dans une course d'éjection, expulsant ainsi du fluide de ladite chambre compressible (604, 612) jusqu'à ladite sortie.

2. Dispositif d'assistance ventriculaire selon la revendication 1, comportant en outre :
un élément de stockage d'énergie (350) destiné à stocker et à libérer de l'énergie de ladite unité de commande électromagnétique sans palier (210, 300, 616) ;
où, pendant la course d'éjection, la puissance électrique délivrée à ladite unité de commande électromagnétique sans palier (210, 300, 616) et l'énergie stockée dans ledit élément de stockage (350) sont délivrées à ladite armature (206, 606, 600) afin de forcer ladite armature en direction de la chambre compressible (204, 612) ;
où ladite armature (206, 606, 600) est découplée de ladite chambre compressible (204, 612) après l'achèvement de ladite course d'éjection de telle façon que l'armature s'escamote dans une course d'escamotage ; et où, à la suite de ladite course d'escamotage, ladite chambre compressible (204, 612) est passivement remplie de sang en provenance de l'entrée (614) et de l'énergie stockée dans ledit élément de stockage (350) au cours de ladite course d'escamotage est délivrée pendant ladite course d'éjection.

3. Dispositif d'assistance ventriculaire selon la revendication 2,
où ledit élément de stockage (350) comprend un ou plusieurs ressorts (319, 320, 330, 340) placés entre ledit cadre (202, 302) et ladite armature (206, 606, 600) afin d'exercer une force de ressort sur ledit cadre (206, 606, 600) ;
où ledit un ou plusieurs aimants (216, 620) génère une force magnétique sur ladite armature (206, 606, 600) résultant de son alimentation électrique et
où ladite une ou plusieurs bobine alimentée (212, 214, 602, 632, 634) génère une force de bobine sur ladite armature (206, 606, 600) qui est approximativement indépendante de la position de ladite armature (206, 606, 600) et qui varie selon le degré d'alimentation de l'une ou plusieurs bobine (212, 214, 602, 632, 634).

4. Dispositif d'assistance ventriculaire selon la revendication 3, où la somme de ladite force de ressort, de ladite force magnétique, et de ladite force de bobine est approximativement indépendante de la position de ladite armature (206, 606, 600) et varie selon le degré d'alimentation de l'une ou plusieurs bobine (212, 214, 602, 632,634).

5. Dispositif d'assistance ventriculaire selon la revendication 3, où ledit ressort est un anneau plat.

6. Dispositif d'assistance ventriculaire selon la revendication 5, où ledit anneau plat se compose de deux ou plusieurs anneaux agencés en couches (410a, 410b, 410c, 410d, 410d).

7. Dispositif d'assistance ventriculaire selon la revendication 6, où lesdites couches (410a, 410b, 410c, 410d, 410d) sont attachées à au moins deux parties mobiles (420, 440) dudit dispositif et à au moins deux parties fixes (430, 450) dudit dispositif, où lesdites couches (410a, 410b, 410c, 410d, 410d) sont couplées les unes aux autres au niveau des fixations (420, 430, 440).

8. Dispositif d'assistance ventriculaire selon la revendication 3, où ledit ressort comporte un ou plusieurs ressorts hélicoïdaux.

9. Dispositif d'assistance ventriculaire selon la revendication 1, comportant en outre un tube percutané d'évacuation vers l'atmosphère pour permettre le remplissage passif de ladite chambre (204, 612).

10. Dispositif d'assistance ventriculaire selon la revendication 1, comportant en outre une chambre de déplacement de volume pour permettre le remplissage de ladite chambre sans évacuation vers l'atmosphère.

11. Dispositif d'assistance ventriculaire selon la revendication 1, où ladite sortie est apte à être couplée avec une aorte et où ladite entrée (614) est apte à être couplée à un ventricule pour permettre le remplissage passif de la chambre (204, 612) à partir dudit ventricule.

12. Dispositif d'assistance ventriculaire selon la revendication 1, où ladite chambre compressible (204, 612) est un sac flexible.

13. Dispositif d'assistance ventriculaire selon la revendication 12, où une plaque mobile (218, 210) est attachée audit sac flexible.

14. Dispositif d'assistance ventriculaire selon la revendication 1, où ladite armature (206, 606, 600) comprend en outre une plaque mobile (218, 610).

15. Dispositif d'assistance ventriculaire selon la revendication 1, où lesdites une ou plusieurs bobine (212, 214, 602, 632, 634) sont positionnées de manière à générer un trajet de flux de bobine à travers des parties magnétiquement perméables de l'armature (206, 606, 600), et où le flux de bobine généré par lesdites une ou plusieurs bobines (212, 214, 602, 632, 634) déplace ladite armature (206, 606, 600) en direction de ladite chambre compressible (204, 612).

16. Dispositif d'assistance ventriculaire selon la revendication 1, où ladite armature (206, 606, 600) est instable dans une position centrale entre ladite unité de commande (210, 300, 616) et ladite chambre (204, 612) ; et où ladite armature (206, 606, 600) est éloignée de ladite chambre (204, 612) lorsque lesdites bobines (212, 214, 602, 632, 634) ne sont pas alimentées.

17. Dispositif d'assistance ventriculaire selon la revendication 1, où un premier parmi lesdits noyaux (222, 224, 604, 608) est sans bobine, et une ou plusieurs desdites bobines (212, 214, 602, 632, 634) sont enroulées autour d'un second parmi lesdits noyaux (222, 224, 604, 608).

18. Dispositif d'assistance ventriculaire selon la revendication 17, où une paire desdites bobines (212, 214, 602, 632, 634) est enroulée autour du second desdits noyaux (222, 224, 604, 608).

19. Dispositif d'assistance ventriculaire selon la revendication 18, où une autre paire desdites bobines (212, 214, 602, 632, 634) est enroulée autour du premier desdits noyaux (222, 224, 604, 608).

20. Dispositif d'assistance ventriculaire selon la revendication 17, où une desdites bobines (212, 214, 602, 632, 634) est enroulée autour du second desdits noyaux (222, 224, 604, 608).

21. Dispositif d'assistance ventriculaire selon la revendication 20, où une autre desdites bobines (212, 214, 602, 632, 634) est enroulée autour du premier desdits noyaux (222, 224, 604, 608).

22. Dispositif d'assistance ventriculaire selon la revendication 17, où ladite chambre (204, 612) est positionnée entre ladite armature (206, 606, 600) et ledit noyau sans bobine (222, 224, 604, 608), et où ladite armature (206, 606, 600) s'escamote en direction dudit second desdits noyaux (222, 224, 604, 608) pendant ladite course d'escamotage.

23. Dispositif d'assistance ventriculaire selon la revendication 1, où une ou plusieurs desdites bobines (212, 214, 602, 632, 634) sont enroulées autour d'un premier desdits noyaux (222, 224, 604, 608), et une ou plusieurs desdites bobines (212, 214, 602, 632, 634) sont enroulées autour d'un second desdits noyaux (222, 224, 604, 608), et où ladite chambre (204, 612) est positionnée entre ladite armature (206, 606, 600) et ledit premier desdits noyaux (222, 224, 604, 608) et où ladite armature (206, 606, 600) s'escamote en direction dudit second desdits noyaux (222, 224, 604, 608) pendant ladite course d'escamotage.
